# EUROPEAN PATENT SPECIFICATION

(11) **EP 3 918 343 B1**
(45) Date of publication and mention of the grant of the patent: **11.06.2025**
(21) Application number: 20701219.6
(22) Date of filing: 27.01.2020
(51) Int. Cl.: G01N 33/74

(54) **METHOD TO PREDICT TREATMENT RESPONSE IN PATIENTS WITH NOCTURNAL ENURESIS**
VERFAHREN ZUR VORHERSAGE DER BEHANDLUNG BEI PATIENTEN MIT ENURESIS NOCTURNA
PROCÉDÉ POUR PRÉDIRE LA RÉPONSE À UN TRAITEMENT DE PATIENTS ATTEINTS D'ÉNURÉSIE NOCTURNE

(30) Priority: 28.01.2019 EP 19154045
(43) Date of publication of application: 08.12.2021
(73) Proprietor: B.R.A.H.M.S GmbH, 16761 Hennigsdorf (DE); Universitätsspital Basel, 4031 Basel (CH)
(72) Inventor: STADE, Katrin, 10779 Berlin (DE); CHRIST-CRAIN, Mirjam, 4031 Basel (CH)
(74) Representative: Vossius & Partner Patentanwälte Rechtsanwälte mbB
(86) International application number: PCT/EP2020/051899
(87) International publication number: WO 2020/156988

(56) References cited:
- EP-A1- 1 628 136
- HARA T. ET AL.: "Evaluation of urinary aquaporin 2 and plasma copeptin as biomarkers of effectiveness of desmopressin acetate for the treatment of monosymptomatic nocturnal enuresis", J. UROL., vol. 198, no. 4, October 2017 (2017-10-01), pages 921 - 927, XP085190620
- NEVÉUS T. ET AL.: "Vasopressin and hypercalciuria in enuresis: a reappraisal", BJU INTERNATIONAL, vol. 90, 2002, pages 725 - 729, XP055574126
- ACETO G. ET AL.: "Enuresis subtypes based on nocturnal hypercalciuria: A multicenter study", J. UROL., vol. 170, no. 4, October 2003 (2003-10-01), pages 1670 - 1673, XP005533133
- BURGU B. ET AL.: "Prospective evaluation of factors affecting the response and relapse rates to desmopressin therapy in male monosymptomatic enuretic adults", UROLOGY, vol. 74, no. 4, October 2009 (2009-10-01), pages 915 - 919, XP026666612
- MORGENTHALER N.G. ET AL.: "Assay for the measurement of copeptin, a stable peptide derived from the precursor of vasopressin", CLIN. CHEM., vol. 52, no. 1, 2006, pages 112 - 119, XP002510790

## Description

### FIELD OF THE INVENTION

The present invention relates to a method for predicting whether a subject to be treated for monosymptomatic nocturnal enuresis will respond to said treatment. The prediction is based on the first level of copeptin in a first sample of the subject at a first time and on the second level of copeptin in a second sample of the subject at a second time.

### BACKGROUND OF THE INVENTION

Nocturnal enuresis (also called bedwetting), is involuntary urination while asleep after the age at which bladder control usually begins. Most children achieve bladder control by ages 4-7. Monosymptomatic nocturnal enuresis (also known as "monosymptomatic enuresis nocturna"; abbreviated MNE or MEN) is a common problem in children, affecting 7-10% of all 7 year olds. MEN is often leading to psychosocial problems because of its burden and stigmatism. The mismatch between the capacity of the bladder to accommodate urine during the night and the nocturnal urine production together with the failure of the child to awake when this happens are the prerequisites for an enuretic episode (Kamperis et al. (2006), Am J Physiol Renal Physiol 291: F1232-F1240).

The only available medical treatment option is the vasopressin analogum Desmopressin. This treatment aims to reduce nocturnal urinary production to a volume less than the functional bladder capacity because enuresis has been shown to occur only when bladder capacity is exceeded (Rittig et al. (1989), J Am Physiol Soc, 256:F664-F671). However, according to the literature, only one third of patients shows a good treatment response, defined as more than 90% of reduced bed wetting (Tauris et al. (2012), J Ped Urol 8:285-290). Despite a consequent therapy with Desmopressin during 4 to 12 weeks with a dose between 20 to 60 µg Desmopressin two third of patients experience a treatment failure with wet nights (defined as Desmopressin non-responders).

Furthermore, treatment with Desmopressin is expensive and may lead to psychosocial pressure in rendering the children ill. Especially the non-responders to therapy suffer from the condition of bed wetting. Furthermore, potential side effects are dangerous like water intoxication and hypertension. Therefore, Desmopressin treatment should be administered only to these patients who have a high chance of a sustainable benefit from Desmopressin treatment. This implies that predictors for treatment success are important and urgently needed to limit Desmopressin therapy to those children with a high chance for good treatment response. In this context, arginine vasopressin (AVP) is the key hormone in regulating water balance and several studies have shown a correlation of low AVP levels at night and nocturnal enuresis as its consequence (Rittig et al. (1989), loc. cit.; Evans and Meadow (1992), Archives of Disease in Childhood, 67:184-188; Nalbantoglu et al. (2013), Urology 82:1120-1124). More specifically, compared with normal subjects, those with enuresis showed a significantly less pronounced diurnal variation with only a slight increase from day to night level of AVP. For example, Rittig et al. (J Urol. 2008; 179(6):2389-95) correlated the circardian rhythm of plasma arginine vasopressin (AVP) to desmopressin response and enuresis status in 78 children 6 to 17 years old. Patients with enuresis and Desmopressin response had the most pronounced arginine vasopressin deficiency during the night compared to subjects without enuresis. Unfortunately, AVP is difficult to measure because of its instability (Robertson (2001), Endocrinol Metab Clin North Am 30:671-94). In this context, determining plasma AVP concentrations by many laboratory assays is not reliable. More than 90 % of AVP in the circulation is bound to platelets and rapidly cleared, resulting in underestimation of AVP levels in case of prolonged storage of unprocessed blood samples. Thus, in pediatrics, measurements of AVP are rarely used clinically. Hara et al. (J Urol. 2017; 198(4):921-927) evaluated the biomarkers urinary aquaporin 2 and plasma copeptin, as a surrogate marker for vasopressin, for predicting desmopressin treatment outcome in 24 children with monosymptomatic nocturnal enuresis and nocturnal polyuria.

Reliable means and methods for predicting whether a subject to be treated for monosymptomatic nocturnal enuresis will respond to said treatment are currently not available.

Therefore, the technical problem underlying the invention is the provision of means and methods for predicting whether a subject to be treated for monosymptomatic nocturnal enuresis will respond to said treatment.

The technical problem is solved by provision of the embodiments provided herein below and as characterized in the appended claims.

### SUMMARY OF THE INVENTION

The present invention relates to a method for predicting whether a subject to be treated for monosymptomatic nocturnal enuresis will respond to said treatment comprising the steps of
(i) determining a first level (c1) of copeptin in a first sample of said subject which has been obtained at a first time,
(ii) determining a second level (c2) of copeptin in a second sample of said subject which has been obtained at a second time,

a) wherein said first time is after an overnight fast of minimum 8 hrs and between 6 a.m. and 9 a.m. in the morning and said second time is between 6 p.m. and 9 p.m. at the same day, and
   wherein if the ratio of said first level to said second level (c1 / c2) is ≥ 1.00, said subject is predicted to respond to said treatment.

In another aspect, the present invention relates to a method for predicting whether a subject to be treated for monosymptomatic nocturnal enuresis will respond to said treatment comprising the steps of
(i) determining a first level (c1) of copeptin in a first sample of said subject which has been obtained at a first time,
(ii) determining a second level (c2) of copeptin in a second sample of said subject which has been obtained at a second time,

a) wherein said first time is after an overnight fast of minimum 8 hrs and between 6 a.m. and 9 a.m. in the morning and said second time is between 6 p.m. and 9 p.m. at the same day, and
   wherein if the difference between said first level and said second level (c1 - c2) is ≥ 0.5 pg/ml, said subject is predicted to respond to said treatment.

In a preferred aspect of the method of the present invention, said treatment is a treatment with Desmopressin or any derivatives thereof.

In a preferred aspect of the method of the present invention, said subject is between 5 and 17 years of age, preferably between 5 and 16 years of age, more preferably between 6 and 16 years of age, more preferably between 6 and 11 years of age.

In a preferred aspect of the method of the present invention, said first and second samples are samples of a bodily fluid, preferably blood, more preferably plasma or serum.

In a preferred aspect of the method of the present invention, said level of copeptin is determined using a method selected from the group consisting of Luminescenceimmunoassay (LIA), radioimmunoassay (RIA), chemiluminescence- and fluorescence- immunoassay, enzyme immunoassay (EIA), Enzyme-linked immunoassay (ELISA), luminescence-based bead array, magnetic beads based array, protein microarray assay, rapid test formats, and rare cryptate assay.

In a preferred aspect of the method of the present invention, the method is an immunoassay comprising the steps of:
a) contacting the first and second samples with
   (i) a first antibody or an antigen-binding fragment or derivative thereof specific for a first epitope of copeptin, and
   (ii) a second antibody or an antigen-binding fragment or derivative thereof specific for a second epitope of copeptin; and
b) detecting the binding of the first and second antibodies or antigen-binding fragments or derivates thereof to copeptin;
optionally wherein the first antibody and the second antibody are present dispersed in a liquid reaction mixture, and wherein a first labelling component which is part of a labelling system based on fluorescence or chemiluminescence extinction or amplification is bound to the first antibody, and a second labelling component of said labelling system is bound to the second antibody so that, after binding of both antibodies to copeptin, a measurable signal which permits detection of the resulting sandwich complexes in the measuring solution is generated.

In one aspect the invention relates to Desmopressin for use in the treatment of monosymptomatic nocturnal enuresis, wherein Desmopressin is administered to a subject that has been determined to be responsive in a method according to the present invention.

### BRIEF DESCRIPTION OF THE DRAWINGS

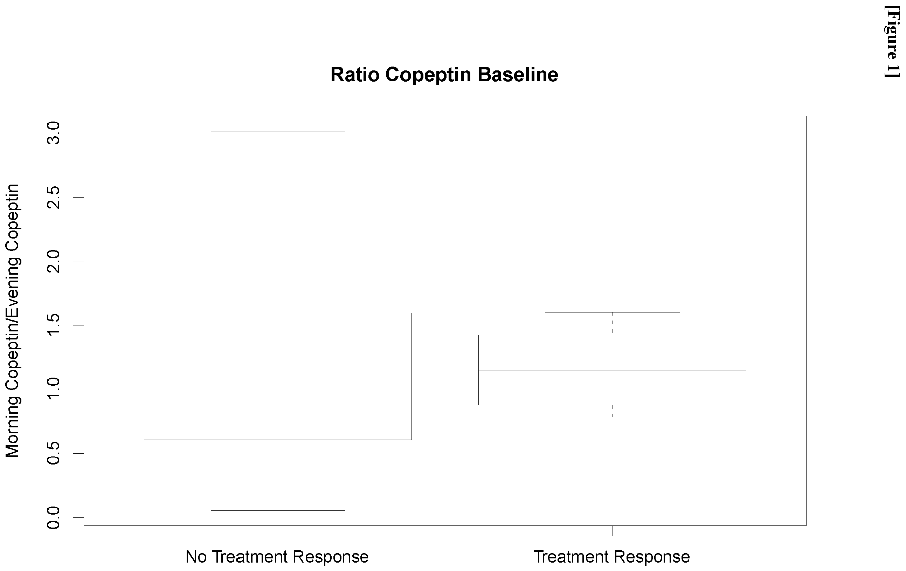

- FIGURE 1:: Change in morning copeptin/evening copeptin ratios in treatment responders and non-responders of samples of subjects to be treated for monosymptomatic nocturnal enuresis is shown by the ratio of the morning copeptin and the evening copeptin level. The first level of the morning copeptin was measured at 8 a.m. and the second level of the evening copeptin was measured between 8 and 10 p.m. at baseline before treatment with Desmopressin. Treatment response (responders) and no treatment response (non-responders) was determined after 4 weeks of treatment with Desmopressin. The median of the ratio (morning copeptin/evening copeptin) of responders and non-responders is shown in Table 2.

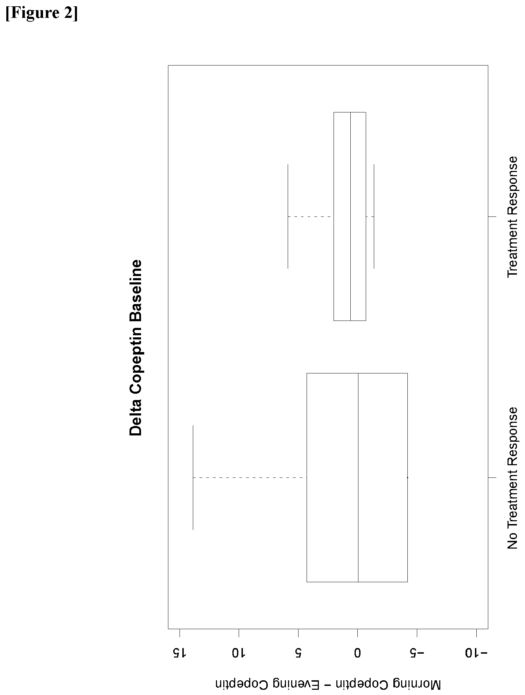

- FIGURE 2:: Change in amplitude morning copeptin-evening copeptin levels in treatment responders and non-responders of samples of subjects to be treated for monosymptomatic nocturnal enuresis is shown by the delta copeptin, i.e. the morning copeptin level minus the evening copeptin level. The first level of the morning copeptin was measured at 8 a.m. and the second level of the evening copeptin was measured between 8 and 10 p.m. at baseline before treatment with Desmopressin. Treatment response (responders) and no treatment response (non-responders) was determined after 4 weeks of treatment with Desmopressin. The median of the delta (morning copeptin-evening copeptin) of responders and non-responders is shown in Table 2.

### DETAILED DESCRIPTION OF THE INVENTION

An object of the invention is to provide an *in vitro* method for predicting whether a subject to be treated for monosymptomatic nocturnal enuresis (MEN) will respond to said treatment, which provides reliable information for the medical practitioner in its decision whether therapy, in particular Desmopressin therapy, in subjects would have a high chance for good treatment response. In this context, according to the literature, only one third of patients show a good treatment response, defined as more than 90% of reduced bed wetting (Tauris et al. (2012), loc. cit.). Thus, means and methods to identify patients having nocturnal enuresis, particularly MEN, and which respond to Desmopressin are needed. Moreover, since Desmopressin may lead to psychosocial problems, high costs and potentially dangerous side effects like water intoxication and hypertension, Desmopressin treatment should be administered only to these patients who have a high chance of a sustainable benefit from Desmopressin treatment.

Desmopressin is 1-deamino-8-D-arginine vasopressin and is usually formulated as an acetate salt. It has several synonyms including 1-(3-mercaptopropionic acid)-8-D-argininevasopressin, 1-desamino-8-D-arginine vasopressin, dDAVP, Desmopresina, Desmopressine and Desmopressinum. Trade names for Desmopressin-containing medicaments include Adin^{®}, Desmogalen^{®}, Desmospray^{®}, Desmotabs^{®}, Minirin^{®}, Nocutil^{®}, Novidin^{®}, Octostim^{®}, Diuretin^{®}, DesmoMelt^{®} and Stimate^{®}. Desmopressin can, e.g., be administered nasally, orally (both preferred) or intravenously as will be further outlined herein below.

The nocturnal enuresis herein is a monosymptomatic nocturnal enuresis (MEN), i.e. a nocturnal enuresis without further symptoms. In particular, the term "monosymptomatic nocturnal enuresis" refers to nocturnal enuresis caused by an insufficient vasopressin release. The monosymptomatic nocturnal enuresis may occur in children with a delayed or incomplete physiological development.

In particular, the subjects do not have daytime enuresis or factors influencing the level of copeptin. Such factors include lower urinary tract symptoms and infection, structural abnormalities of the urinary tract, chronic illness, esp. renal failure, hypertension, congenital heart disease, diabetes mellitus and diabetes insipidus. The terms "monosymptomatic nocturnal enuresis", "monosymptomatic enuresis noctuma, "MEN" and "MNE" are used synonymously herein.

The present invention is based on the surprising finding that there is a relation between the magnitude of the withinday amplitude of copeptin level in a sample of a subject to be treated for MEN, and the responsiveness of said subject to a medical treatment. In this context, a sensitive and precise assay comprising measuring the level of copeptin, in order to predict whether a subject to be treated for MEN will respond to said treatment is provided by the present invention. Subject of the present invention is thus an *in vitro* method for predicting whether a subject to be treated for MEN will respond to said treatment. A further subject of the present invention is a sensitive and precise copeptin assay. In the appended examples, the results of a clinical study are documented. This clinical study demonstrates that the magnitude withinday amplitude of copeptin at baseline has an unexpectedly strong relationship with the subject's response to treatment with Desmopressin. Moreover, the study indicated that the calculated difference or ratio of a first and a second level of the withinday copeptin at baseline predicts the chance of successful treatment, reflected by Desmopressin treatment.

More in particular, the present invention pertains to a method for predicting whether a subject to be treated for MEN will respond to said treatment, wherein the method comprises (i) determining a first level (c1) of copeptin in a first sample of said subject which has been obtained at a first time, and (ii) determining a second level (c2) of copeptin in a second sample of said subject which has been obtained at a second time.

Typically, said first level of copeptin is obtained after an overnight fast of minimum 8 hrs and between 6 a.m. and 9 a.m. in the morning and said second level of copeptin is obtained between 6 pm and 9 p.m. at the same day. All times are according to the local time zone of the location in which the subject lives. Herein, if the ratio of said first level to said second level (c1 / c2) is ≥ 1.00, said subject is predicted to respond to said treatment. In particular, if a difference between said first level and said second level (c1 - c2) is ≥ 0.5 pg/ml, said subject is predicted to respond to said treatment Thereby, said amplitude may be measured at baseline before treatment or during or after the treatment has been initiated. Preferably, said amplitude may be measured at baseline before treatment.

As disclosed herein and illustrated in the appended Examples, said first and said second level of copeptin can be measured at baseline before treatment. Thus, in the context of the method of the invention, said first and said second level of copeptin may be determined in samples of a subject to be treated for MEN, before onset of said treatment. The subject may only be treated for MEN, if the subject has been predicted to respond to the treatment, e.g. if the ratio of said first level obtained after an overnight fast of minimum 8 hrs and between 6 a.m. and 9 a.m. to said second level obtained between 6 p.m. and 9 p.m. at the same day (c1 / c2) is ≥ 1.00. As another example, the subject may only be treated for MEN, if the subject has been predicted to respond to the treatment, e.g, if a difference between said first level obtained after an overnight fast of minimum 8 hrs and between 6 a.m. and 9 a.m and said second level obtained between 6 pm and 9 p.m. at the same day (c1 - c2) is ≥ 0.5 pg/ml.

The first and second level of copeptin can be determined by methods known in the art as will be exemplarily outlined herein below in more detail.

The functional assay sensitivity for the detection of copeptin level may be particularly below 5 pg/ml, more preferably 4 pg/ml or below, even more preferred 3 pg/ml or below, most preferred 2 pg/ml or below. For example, the functional sensitivity may be 4.34 pg/ml. In one embodiment, the functional assay sensitivity for the detection of copeptin level may be 4 pg/ml or below. In another embodiment, the functional assay sensitivity for the detection of copeptin levelmay be 3 pg/ml or below. The functional assay sensitivity (FAS) is defined as the lowest concentration of an assay that can be measured with an interassay coefficient variance (CV) of 20 % (Spencer CA. 1989. J Clin Immunoassay 12: 82-9). Such a low functional assay sensitivity may allow for the correlation of copeptin level with the prediction whether a subject to be treated for monosymptomatic nocturnal enuresis will respond to said treatment. For example, a low functional assay sensitivity may allow prediction whether a subject to be treated for monosymptomatic nocturnal enuresis will respond to said treatment, wherein a first and a second level of copeptin is measured before said treatment.

The limit of detection of the assay for the detection of copeptin level may be particularly below 100 pg/ml, preferably 10 pg/ml or below, even more preferred 3.00 pg/ml or below, most preferred 2.77 pg/ml or below. The limit of detection (LOD) is defined as the lowest quantity or concentration of a component that can be reliably detected with a given analytical method (MacDougall et al. 1980. Analytical Chemistry 52:2242-49). Such a low limit of detection may allow for the correlation of copeptin level with the prediction whether a subject to be treated for monosymptomatic nocturnal enuresis will respond to said treatment. For example, a low limit of detection may allow prediction whether a subject to be treated for monosymptomatic nocturnal enuresis will respond to said treatment, wherein a first and a second level of copeptin is measured before said treatment.

As another example, a low functional assay sensitivity as described herein and a low limit of detection as described herein may allow prediction whether a subject to be treated for monosymptomatic nocturnal enuresis will respond to said treatment, wherein a first and a second level of copeptin is measured before said treatment.

For example, an immunofluorescent assay may be used for the detection of copeptin level. Immunofluorescent assays that may be used in the method of the present invention may include but is not restricted to the Thermo Scientific^{™} B·R·A·H·M·S ^{™} Copeptin pro AVP immunoflourescent assay. Such assays have been described, for example, in Morgenthaler et al. 2008. Trends in Endocrinology and Metabolism 19(2):43-9; Möckel et al. 2015. Eur Heart J. 36(6): 369-376; Timper et al. 2015. J Clin Endocrinol Metab. 100(6):2268-74; Roffi et al. 2015. Eur Heart J. and Winzeler et al. 2015. J Clin Endocrinol Metab. http://dx.doi.org/10.1210/jc.2014-4527.

The term "predicting whether a subject to be treated for monosymptomatic nocturnal enuresis will respond to said treatment" as used in accordance with the method of the present invention refers to identifying a chance of a patient with monosymptomatic nocturnal enuresis during and/or after the treatment to respond to said treatment. This may also include an estimation of the chance of recovery (responder) or the chance of no recovery for said subject (no responder). In this context, the condition or disease state of MEN during and/or after the treatment may be compared to the disease state before the treatment. Methods to determine the disease state of MEN are well known in the art and include but are not limited to the determination of the number of bed-wetting nights and/or the number of bed-wettings per night.

The response may range from partial alleviation of the symptoms to a complete remission of the symptoms, indicated by the change of symptoms strength and/or frequency of relapse of individual symptoms and/or the change of the copeptin level as described herein. For example, the response to the treatment is indicated by a reduction in bed-wetting. In one aspect of the present invention, the subject to be treated for MEN responds to the treatment if the number of wet nights per week is reduced by at least 70 %, preferably at least 80 %, more preferably at least 90 %. In another aspect of the present invention, the subject to be treated for MEN responds to the treatment if said subject has at least 10, preferably at least 12, more preferably at least 14 consecutive dry nights.

The response can occur shortly after treatment or after a certain time period. For example, the response can occur after 4 weeks of treatment. In another Example, the response may occur after 12 weeks of treatment. In one aspect of the present invention, a response in a patient with MEN occurs after 4 weeks of treatment. Preferably, a response in a patient with MEN occurs after 12 weeks of treatment. More preferably, the subject to be treated for MEN responds to the treatment if the number of wet nights per week is reduced by 90 % after 4 weeks of treatment. In another aspect of the present invention, the subject to be treated for MEN responds to the treatment if said subject has 14 consecutive dry nights after 4 weeks of treatment. Even more preferably, the subject to be treated for MEN responds to the treatment if the number of wet nights per week is reduced by 90 % after 12 weeks of treatment. In another aspect of the present invention, the subject to be treated for MEN responds to the treatment if said subject has 14 consecutive dry nights after 12 weeks of treatment.

The prediction whether a subject to be treated for MEN will respond to the treatment can be assessed by the determination of a threshold or cut-off value of the difference between said first level and said second level copeptin or the ratio of said first level to said second level. Hereby, the respective cut-off value for predicting whether a subject to be treated for MEN will respond to the treatment refers to measurements of copeptin in one or more sample(s) of the subject or subjects, wherein the subject will respond or not respond to the treatment.

All values disclosed herein relating to the difference between the first level and the second level of a marker or biomarker, such as copeptin, or the ratio of said first level to said second level, are to be understood as "equal or above" a certain cut-off or "below" a certain cut-off. For example, an aspect relating to the difference between the first level and the second level of copeptin of at least 0.5 is to be understood as relating to a level of copeptin equal or above the cut-off value of 0.5. Conversely, an embodiment relating to a level of copeptin below 0.5 is to be understood as relating to a level of copeptin below a cut-off value of 0.5.

Cut-off values of the difference between the first level and the second level of a marker or biomarker, such as copeptin, or the ratio of said first level to said second level, of subjects to be treated for MEN may be determined by previously described methods known in the art. For example, methods are known to a skilled person for using the Coefficient of variation in assessing variability of quantitative assays in order to establish cut-off values (Reed et al., Clin Diagn Lab Immunol.2002; 9(6):1235-1239).

In one aspect of the present invention, a cut-off value may be determined from a subject to be treated for MEN that will respond to the treatment. Preferably, a cut-off value may be determined from a population of subjects to be treated for MEN that will respond to the treatment. More preferably, a cut-off value may be determined by the median of a population of subjects to be treated for MEN that will respond to the treatment. In this case, a sample of the subject to be treated for MEN will respond to the treatment if the difference between said first level and said second level of copeptin, or the ratio of said first level to said second level is equal or above said cut-off value. Conversely, a sample of the subject to be treated for MEN will not respond to the treatment if the difference between said first level and said second level copeptin, or the ratio of said first level to said second level is below said cut-off value.

Population averages levels of copeptin may also be used as cut-off levels, whereby the difference between the first level and the second level of a marker or biomarker, such as copeptin, or the ratio of said first level to said second level of a subject to be treated for MEN may be compared to a cut-off value from a control population, wherein the control group preferably comprises more than 10, 20, 30, 40, 50 or more subjects to be treated for MEN.

In one aspect of the present invention, a cut-off value may be determined from a subject to be treated for MEN, that will not respond to the treatment. In this case, a sample of the subject to be treated for MEN will not respond to the treatment if the difference between said first level and said second level of copeptin or the ratio of said first level to said second level is below said cut-off value. Conversely, a sample of the subject to be treated for MEN will respond to the treatment if the difference between said first level and said second level of copeptin, or the ratio of said first level to said second level is equal or above said cut-off value.

Additionally, functional assay sensitivity can be determined in order to indicate statistically significant values for use as reference levels or cut-offs according to established techniques. Laboratories are capable of independently establishing an assay's functional sensitivity by a clinically relevant protocol. "Functional sensitivity" can be considered as the concentration that results in a coefficient of variation (CV) of 20% (or some other predetermined % CV), and is thus a measure of an assay's precision at low analyte levels. The CV is therefore a standardization of the standard deviation (SD) that allows comparison of variability estimates regardless of the magnitude of analyte concentration, at least throughout most of the working range of the assay.

Furthermore, methods based on ROC analysis can be used to determine statistically significant differences between two clinical patient groups. Receiver Operating Characteristic (ROC) curves measure the sorting efficiency of the model's fitted probabilities to sort the response levels. ROC curves can also aid in setting criterion points in diagnostic tests. The higher the curve from the diagonal, the better the fit. If the logistic fit has more than two response levels, it produces a generalized ROC curve. In such a plot, there is a curve for each response level, which is the ROC curve of that level versus all other levels. Software capable of enabling this kind of analysis in order to establish suitable reference levels and cut-offs is available, for example JMP 12, JMP 13, Statistical Discovery, from SAS.

Cut-off values may similarly determined for the difference between the first level and the second level of a marker or biomarker, such as copeptin, or the ratio of said first level to said second level. Literature is available to a skilled person for determining an appropriate cut-off, for example, Terence Chan et al. (Terence Chan et al. (Expert Rev.Mol.Diagn.2011; 11(5), 487.496) described that indicators such as the positive and negative likelihood ratios, which are calculated based on sensitivity and specificity, are also useful for assessing the strength of a diagnostic test. Values are commonly graphed for multiple cut-off values (CVs) as a receiver operating characteristic curve. The area under the curve value is used to determine the best diagnostically relevant CV. This literature describes the variation of CVs (cut-off values, that is dependent on the assay and study design), and suitable methods for determining cut-off values. The cut-off values may aid in setting criterion points in diagnostic tests.

The sensitivity and specificity of a diagnostic and/or prognostic test depends on more than just the analytical "quality" of the test, they also depend on the definition of what constitutes an abnormal result. In practice, Receiver Operating Characteristic curves (ROC curves), can be calculated by plotting the value of a variable versus its relative frequency in "responder" and "non-responder" populations. For any particular marker (like copeptin), a distribution of marker levels for responder and non-responder subjects will likely overlap. Under such conditions, a test does not absolutely distinguish responder from non-responder with 100% accuracy, and the area of overlap might indicate where the test cannot distinguish responder from non-responder. A threshold is selected, below which the test is considered to identify a "non-responder" and above which the test is considered to identify a "responder" or below or above which the test indicates another specific condition. The area under the ROC curve is a measure of the probability that the perceived measurement will allow correct identification of a condition. ROC curves can be used even when test results do not necessarily give an accurate number. As long as one can rank results, one can create a ROC curve. For example, results of a test on "non-responder" samples might be ranked according to degree (e.g. 1=low, 2=normal, and 3=high). This ranking can be correlated to results in the "responder" population, and a ROC curve created. These methods are well known in the art; see, e.g., Hanley et al. 1982. Radiology 143: 29-36. Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a condition. Thus, the area under the ROC curve can be used to determine the effectiveness of the test.

In the context of the present invention, the difference between the first level and the second level of a marker or biomarker, such as copeptin, or the ratio of said first level to said second level at which or above which it can be predicted whether a subject to be treated for MEN will respond to the treatment or not can be understood as the positive and negative likelihood ratios. Thus, a positive likelihood is to be understood as a subject, which will respond to the treatment. In turn, a negative likelihood is to be understood as a subject which will not respond to the treatment. Thus, a value at which the subject to be treated with MEN will respond to the treatment if said ratio or value is equal or above a certain cut-off value.

The values of the protein level of copeptin disclosed herein refer preferably to measurements in a sample obtained from a subject to be treated MEN. Accordingly, the values disclosed herein may vary to some extent depending on the detection/measurement method employed or vary due to different automated systems, and the specific values disclosed herein are intended to also read on the corresponding values determined by other methods.

Herein, in particular aspects of the invention the subject to be treated for MEN is predicted to respond to said treatment when the difference between said first level and said second level of copeptin is at least 0.5 pg/ml, at least 0.6 pg/ml, at least 0.7 pg/ml, or at least 0.8 pg/ml. Alternatively, the subject to be treated for MEN is predicted to respond to said treatment when the ratio of said first level to said second level (c1 / c2) is at least 0.5, at least 0.7, at least 0.8, at least 1.0, at least 1.1, at least 1.2, or at least 1.3.

In one aspect of the method according to the invention, the subject to be treated for MEN is not treated (yet). In this context, the first level of copeptin is obtained after an overnight fast of minimum 8 hrs and between 6 a.m. and 9 a.m. in the morning and said second level of copeptin is obtained between 6 p.m. and 9 p.m. at the same day, wherein the treatment may be initiated immediately after the second level of copeptin is obtained from the subject. Preferably, the treatment is initiated 0 to 48 hours after the second level of copeptin is obtained. For example, the treatment may be initiated 48 hours after the second level of copeptin is obtained.

In another aspect of the method according to the invention, the subject to be treated for MEN, wherein said subject is predicted to respond to said treatment, is (already) treated. In this context, the first and the second level copeptin is obtained 4 weeks after treatment has been initiated. Preferably, the first and the second level of copeptin is obtained 12 weeks after treatment has been initiated. More preferably, the first and the second level of copeptin is obtained between 4 and 12 weeks after treatment has been initiated. In this case, an already conducted treatment can be continued or the therapy can be adapted based on the difference between or ratio of said first level and said second level of copeptin. It is to be understood that a therapy can be adapted by any conventional methods for adapting a therapy known in the art. For example but not limited thereto, a therapy may be adapted by increasing the dose of the drug for the medical treatment and/or by shorter intervals of drug administration. Preferably, the dose of the drug administered to the subject to be treated for MEN is increased.

In another aspect of the method according to the invention, the subject to be treated for MEN, wherein said subject is predicted not to respond to said treatment, is (already) treated. In this case, an already conducted treatment can be stopped based on the difference between or ratio of said first level and said second level of copeptin.

As used in accordance with the methods of the present invention, the subject is a human child, teenager or young adult. More preferably, the subject is between 5 and 17 years of age, preferably between 5 and 16 years of age, more preferably between 6 and 16 years of age, more preferably between 6 and 11 years of age.

The term "monosymptomatic nocturnal enuresis" refers to any bed-wetting episode in children that occurs in discrete amounts during sleep. According to the International Childern's Continence Society, nocturnal enuresis is defined as any bed-wetting episode in children over 5 years that occurs in discrete amounts during sleep. In particular, Enuresis is divided into monosymptomatic and non-monosymptomatic forms. Monosymptomatic enuresis is defined as enuresis in children without any other lower urinary tract symptoms and without a history of bladder dysfunction. As used herein, a "subject to be treated for monosymptomatic nocturnal enuresis" means that the subject or patient has been diagnosed with MEN. Preferably, the subject has been diagnosed with MEN in the absence of any other lower urinary tract symptom, e.g. increased or decreased frequency of micturition, daytime urinary incontinence, urgency, hesitancy, straining, a weak urinary stream, intermittency, voiding postponement maneuvers, a feeling of incomplete micturition, post-micturition dribble and genital or lower urinary tract pain. The combination of any of these symptoms and enuresis defines the enuresis as non-monosymptomatic. Monosymptomatic nocturnal enuresis usually is divided into primary and secondary forms. Children who have never achieved a satisfactory period of nighttime dryness have primary enuresis. An estimated 80 percent of children with nocturnal enuresis have this form. Children who develop enuresis after a dry period of at least six months have secondary enuresis. Secondary enuresis is often ascribed to an unusually stressful event (e.g., parental divorce, birth of a sibling) at a time of vulnerability in a child's life. Stool retention and suboptimal daytime voiding habits often play a role (Nevéus et al. (2006), J Urol 176:314.; von Gontard et al. (1999), Pediatr Nephrol 13:662). Methods for diagnosis of enuresis and in particular MEN, are well known in the art and include but are not limited to urinalysis, physical examination and frequency of bed-wetting. Term as "diagnosis" is used in this specification as a general term, which, if not defined otherwise, are intended to comprise not only diagnosis in the sense of identifying a specific condition, but also screening of asymptomatic or high risk populations at risk of certain conditions or suspected to have certain conditions, especially for early detection, monitoring of untreated or treated patients and monitoring the course of a therapy, prognosis and for the therapy guidance of untreated or treated patients, especially for the identification of responder or non-responder to a certain treatment.

As used herein, the term "sample" is a biological sample that is obtained or isolated from the subject. "Sample" as used herein may, e.g., refer to a sample of bodily fluid or tissue, urine or saliva obtained for the purpose of predicting if the subject to be treated for MEN will respond to the treatment. Herein said sample is a sample of a bodily fluid, preferably blood, plasma, serum, more preferably plasma or serum. In preferred aspects of the present invention, the level of copeptin is determined in the sample, wherein said sample is a blood, serum or plasma sample. In most preferred aspects, the marker is determined in a plasma sample. In addition, one skilled in the art would realize that some test samples would be more readily analyzed following a fractionation or purification procedure, for example, separation of whole blood into serum or plasma components.

"Plasma" in the context of the present invention is the virtually cell-free supernatant of blood containing anticoagulant obtained after centrifugation. Exemplary anticoagulants include calcium ion binding compounds such as EDTA or citrate and thrombin inhibitors such as heparinates or hirudin. Cell-free plasma can be obtained by centrifugation of the anticoagulated blood (e.g. citrated, EDTA or heparinized blood), for example for at least 15 minutes at 2000 to 3000 g.

"Serum" in the context of the present invention is the liquid fraction of whole blood that is collected after the blood is allowed to clot. When coagulated blood (clotted blood) is centrifuged serum can be obtained as supernatant.

The methods according to the present invention are especially well suited for the prediction whether a subject to be treated for MEN will respond to the treatment. As used herein, "the difference between said first level and said second level" or "the ratio of said first level to said second level" used for prediction whether a subject to be treated for MEN will respond to the treatment may have the meaning of "correlating "the difference between said first level and said second level" or "correlating the ratio of said first level to said second level" with the prediction whether a subject to be treated for MEN will respond to the treatment. In particular, the difference between said first level and said second level refers to the first level of copeptin in the morning minus the second level of copeptin in the evening, wherein the first and the second levels are determined on the same day. Said first level of copeptin is obtained after an overnight fast of minimum 8 hrs and between 6 a.m. and 9 a.m. in the morning and said second level of copeptin is obtained between 6 p.m. and 9 p.m. at the same day.

Furthermore, the ratio of said first level to said second level refers to the first level of copeptin in the morning divided by the second level of copeptin in the evening, whereas the first and the second levels are determined on the same day.

Said first level of copeptin is obtained after an overnight fast of minimum 8 hrs and between 6 a.m. and 9 a.m. in the morning and said second level of copeptin is obtained between 6 p.m. and 9 p.m. at the same day.

In one aspect, the method of the present invention relates to the ratio of said first level to said second level (c1 / c2) of ≥ 1.00 for predicting that a subject to be treated with MEN will respond to said treatment.

In a particular aspect, the method of the present invention relates to a difference between said first level and said second level (c1 - c2) of ≥ 0.5 pg/ml for predicting that a subject to be treated with MEN will respond to said treatment.

Thereby, said levels may be measured at baseline before treatment or during or after the treatment has been initiated. Preferably, said levels may be measured at baseline before treatment. For example, said levels may be measured at baseline on the same day of treatment or at least the second level may be measured at baseline on the same day of treatment. Preferably, said levels may be measured at baseline 0 to 48 hours before treatment. For example, said level may be measured at baseline 48 hours before treatment.

"Prepro-AVP" refers to a precursor protein encoded by the vasopressin gene and comprising a 19 amino acid signal sequence, arginine vasopressin and two associated proteins, neurophysin II and a glycopeptide, copeptin. Arginin Vasopressin (AVP), also known as antidiuretic hormone (ADH), is produced in the hypothalamus and released from the neuropituitary gland in conditions of high plasma osmolality, low plasma volume and low blood pressure. AVP binds to three different receptors (V1aR, V1bR and V2R). The V1 aR is widely expressed (Nature 1993:356:523-526), whereas V1bR and V2R are more specifically expressed in the pituitary gland and kidney collecting ducts, respectively (Nature 1992:336-339 and FEBS Lett 1994:356:215-220). Prepro-AVP consists of the sequence SEQ ID NO.: 1.

AVP consists of the sequence SEQ ID NO.: 2.

Neurophysin has been described as a marker for nicotine uptake (Robinson A. G., Isolation, assay, and secretion of individual human neurophysins, J Clin Invest 1975; 55: 360-367), malignancy- and nonmalignancy-related syndrome of inappropriate ADH secretion (SIADH), and nephrogenic diabetes insipidus (Pullan P. T., Clappison B. H., Johnston C. I., Plasma vasopressin and human neurophysins in physiological and pathological states associated with changes in vasopressin secretion, J Clin Endocrinol Metab 1979; 49: 580-587; North W. G., LaRochelle F. T., Jr., Melton J., Mills R. C., Isolation and partial characterization of two human neurophysins: their use in the development of specific radioimmunoassays. J Clin Endocrinol Metab 1980; 51: 884-891). Neurophysin II consists of the sequence SEQ ID NO.: 4.

The invention relates to a method for predicting whether a subject to be treated with MEN will respond to said treatment, wherein the marker copeptin is determined in the sample of said subject. Copeptin is a biologically inactive cleavage product of the C-terminus of the AVP precursor and is produced in equimolar amounts with AVP. However, in contrast to AVP, copeptin is stable, has a long half-life, is not bound to platelets and therefore found in considerably higher concentrations in plasma than AVP. Copeptin was found to be a stable and sensitive marker for AVP release. It seems to be released stoichiometrically together with AVP and is stable for days at room temperature and can be detected within 3 hours (Robertson (2001), loc. cit.). In this context, copeptin, mirroring AVP, has been shown to be significantly lower in patients with MEN, compared to controls and lower in patients with severe bed-wetting compared to patients with only slight bed-wetting. In one aspect, the invention relates to a method for predicting whether a subject to be treated with MEN will respond to said treatment, wherein the marker copeptin or a fragment thereof is determined in the sample of said subject. Preferably, copeptin or a fragment thereof as used in the method of the present invention comprises the sequence SEQ ID NO.: 3 (fragment: amino acids 126-164 of prepro-AVP). More preferably, copeptin or a fragment thereof as used in the method of the present invention essentially consists of the sequence SEQ ID NO.: 3. Even more preferably, copeptin or a fragment thereof as used in the method of the present invention consists of the sequence SEQ ID NO.: 3. These polypeptides according to the invention may also have post-translational modifications such as glycolization, lip(o)idization, or derivatization.

It is also envisaged herein that the level of a copeptin polypeptide is determined that has a sequence identity of at least 75%, for example, 75%, 80%, 85%, 90%, 91%, 92%, 93%, 94%, 95%, 96%, 97%, 98% or 99% sequence identity as shown in SEQ ID NO: 3, wherein the higher values of sequence identity are preferred. In accordance with the present invention, the terms "sequence identity", "homology" or "percent homology" or "identical" or "percent identity" or "percentage identity" in the context of two or more amino acid sequences refers to two or more sequences or subsequences that are the same, or that have a specified percentage of amino acids that are the same, when compared and aligned for maximum correspondence over the window of comparison (preferably over the full length), or over a designated region as measured using a sequence comparison algorithm as known in the art, or by manual alignment and visual inspection. Sequences having, for example, 70% to 90% or greater sequence identity may be considered to be substantially identical. Such a definition also applies to the complement of a test sequence. Preferably, the described identity exists over a region that is at least about 5 to about 8 amino acids in length, more preferably, over a region that is at least about 25 to about 35 amino acids in length, even more preferably, over a region that is at least about 65 to about 83 amino acids in length, even more preferably, over a region that is at least about 115 to about 148 amino acids in length, most preferably, over the full length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

As used herein, the term "the level of copeptin" refers to the quantity of the molecular entity of the marker copeptin in a sample that is obtained from the subject. In other words, the concentration of the marker is determined in the sample. Hence, the term "the level of copeptin" refers to the quantity of the molecular entity of the marker copeptin in a sample that is obtained from the subject. As described above, it is also envisaged herein that a fragment of copeptin can be detected and quantified. The fragment can have any length, e.g. at least about 5, 10, 20, 30, 40, 50 or 100 amino acids, so long as the fragment allows the unambiguous determination of the level of copeptin. The level of copeptin is determined.

The determination of copeptin also encompasses measuring and/or detecting specific subregions of these molecules, for example by employing antibodies or other affinity reagents directed against a particular portion of the molecules, or by determining the presence and/or quantity of the molecules by measuring a portion of the protein using mass spectrometry. In a preferred aspect of the present invention, the level of copeptin is determined in an assay, preferably a diagnostic assay. As mentioned herein, an "assay" or "diagnostic assay" can be of any type applied in the field of diagnostics. Such an assay may be based on the binding of an analyte to be detected to one or more capture probes with a certain affinity. In the context of the present invention, "capture molecules" are molecules which may be used to bind target molecules or molecules of interest, i.e. analytes (i.e. in the context of the present invention the copeptin from a sample). Capture molecules must thus be shaped adequately, both spatially and in terms of surface features, such as surface charge, hydrophobicity, hydrophilicity, presence or absence of lewis donors and/or acceptors, to specifically bind the target molecules or molecules of interest. Hereby, the binding may for instance be mediated by ionic, van-der-Waals, pi-pi, sigma-pi, hydrophobic or hydrogen bond interactions or a combination of two or more of the aforementioned interactions between the capture molecules and the target molecules or molecules of interest. In the context of the present invention, capture molecules may for instance be selected from the group comprising a nucleic acid molecule, a carbohydrate molecule, a RNA molecule, a protein, an antibody, a peptide or a glycoprotein. Preferably, the capture molecules are antibodies, including fragments thereof with sufficient affinity to a target or molecule of interest, and including recombinant antibodies or recombinant antibody fragments, as well as chemically and/or biochemically modified derivatives of said antibodies or fragments derived from the variant chain with a length of at least 12 amino acids thereof. Suitable methods to determine the level of copeptin are described herein below in detail.

In addition to the level of copeptin further parameters of the subject may be determined such as further markers, clinical scores and/or further clinical parameters. Hence copeptin can be part of a marker panel. Thus, the prediction whether a subject to be treated for MEN will respond to the treatment may in one embodiment be improved by additionally determining and using at least one laboratory parameter or further marker selected from the group consisting of functional bladder volume, sleep fragmentation, assessment of periodic limb movement disorder (PLMD) signs such as involuntary movements of the limbs, flexion in the knee, hip, or ankle during non-rapid eye movement phase of sleep, cardiac rate, blood pressure, diastolic blood pressure, systolic blood pressure, age, gender, functional bladder volume, the level of prolactin-inhibiting hormone, the level of neurophysin, the level of vasopressin, the level of prostaglandin, the level of aquaporin 1, the level of aquaporin 2, the level of aquaporin 3, the level of aquaporin 4, the level of proadrenomedullin and fragments thereof, and the level of pro-endothelin-1 and fragments thereof.

As used herein, terms such as "marker", "surrogate", "prognostic marker", "factor" or "biomarker" or "biological marker" are used interchangeably and relate to measurable and quantifiable biological markers (e.g., specific enzyme concentration or a fragment thereof, specific hormone concentration or a fragment thereof, or presence of biological substances or a fragment thereof) which serve as indices for health- and physiology-related assessments, such as a disease/disorder/clinical condition risk. Furthermore, a biomarker is defined as a characteristic that is objectively measured and evaluated as an indicator of normal biological processes, pathogenic processes, or pharmacologic responses to a therapeutic intervention. A biomarker may be measured on a biological sample (as a blood, plasma, urine, or tissue test).

As used herein, a parameter is a characteristic feature, or measurable factor that can help in defining a particular system. A parameter is an important element for health- and physiology-related assessments, such as a disease/disorder/clinical condition risk. Furthermore, a parameter is defined as a characteristic that is objectively measured and evaluated as an indicator of normal biological processes, pathogenic processes, or pharmacologic responses to a therapeutic intervention.

Thus, the determined level copeptin in a samples of subjects may be used either alone or in conjunction with other prognostically useful laboratory or clinical parameters for the prediction whether a subject to be treated for MEN, will respond to the treatment.

According to the present invention, level of copeptin and/or other markers or parameters are employed as markers and parameters for predicting if a subject to be treated for MEN will respond to the treatment. A skilled person is capable of obtaining or developing means for the identification, measurement, determination and/or quantification of the copeptin as well as the other markers or parameters as outlined above according to standard molecular biological practice. The level of copeptin as well as the other markers or parameters can be determined by any assay that reliably determines the concentration of the marker.

The level of copeptin and/or further markers and/or parameters may be determined by an immunoassay. As used herein, an immunoassay is a biochemical test that measures the presence or concentration of a macromolecule/polypeptide in a solution through the use of an antibody or antibody binding fragment or immunoglobulin.

Immunoassays in various formats such as for instance sandwich, enzyme-linked immunosorbent assay, luminescent immunoassay, rapid test formats, assays suitable for point-of-care testing and homogeneous assays such as, for example, the Kryptor system (BRAHMS/Thermo Fisher Scientific) can be employed. In a preferred embodiment, the level of copeptin or fragments thereof having at least a length of 12 amino acids is determined. In the context of the present invention, said level of copeptin is determined using a method selected from the group consisting of Luminescenceimmunoassay (LIA), radioimmunoassay (RIA), chemiluminescence- and fluorescence- immunoassay, enzyme immunoassay (EIA), Enzyme-linked immunoassay (ELISA), luminescence-based bead array, magnetic beads based array, protein microarray assay, rapid test formats, and rare cryptate assay. The assay can be performed in homogeneous phase or in heterogeneous phase as further outlined herein below. Preferred detection methods comprise immunoassays in various formats such as for instance radioimmunoassays, chemiluminescence- and fluorescence- immunoassays, Enzyme-linked immunoassays (ELISA), Luminex-based bead arrays, protein microarray assays, assays suitable for point-of-care testing and rapid test formats such as for instance immunechromatographic strip tests. Such an assay may be based on the binding of an analyte to be detected to one or more capture probes with a certain affinity.

Determination of copeptin and optional other markers based on antibody recognition is a preferred aspect of the present invention. As used herein, the term, "antibody" refers to immunoglobulin molecules and immunologically active portions of immunoglobulin (Ig) molecules, i.e., molecules that contain an antigen binding site that specifically binds (immunoreacts with) an antigen. According to the invention, the antibodies may be monoclonal as well as polyclonal antibodies. Preferably, antibodies may be monoclonal. Particularly, antibodies that are specifically binding to copeptin are used. An antibody is considered to be specific, if its affinity towards the molecule of interest, e.g. copeptin, is at least 50-fold higher, preferably 100-fold higher, most preferably at least 1000-fold higher than towards other molecules comprised in a sample containing the molecule of interest. It is well known in the art how to develop and to select antibodies with a given specificity. In the context of the invention, monoclonal antibodies are preferred. The antibody or the antibody binding fragment binds specifically to the herein defined markers or fragments thereof. In particular, the antibody or the antibody binding fragment binds to the herein defined peptides of copeptin. Thus, the herein defined peptides can also be epitopes to which the antibodies specifically bind. Further, an antibody or an antibody binding fragment is used in the methods of the invention that binds specifically to copeptin. Further, an antibody or an antibody binding fragment is used in the methods of the invention that binds specifically to copeptin and optionally to other markers of the present inventions.

The method according to the present invention is particularly preferred, wherein copeptin is employed for the determination of copeptin in a sample. In certain aspects, the level of the marker is determined by high performance liquid chromatography (HPLC). In certain aspects, the HPLC can be coupled to an immunoassay. In certain aspects of the present invention, copeptin is determined in a sandwich immunoassay. In this sandwich immunoassay, two antibodies are applied for, e.g., one marker such as copeptin in a sample. In particular, this is preferred, if copeptin is determined by the use of two antibodies, which specifically bind to different partial sequences of copeptin or a fragment thereof.

In a preferred aspect of the *in vitro* method according the invention, one of the antibodies is labeled and the second one is bound to or may be bound selectively to a solid phase.

In a particularly preferred aspect of the assay, one of the antibodies is labeled while the other is either bound to a solid phase or can be bound selectively to a solid phase. In a preferred embodiment the method is executed as heterogeneous sandwich immunoassay, wherein one of the antibodies is immobilized on an arbitrarily chosen solid phase, for example, the walls of coated test tubes (e.g. polystyrol test tubes; coated tubes; CT) or microtiter plates, for example composed of polystyrol, or to particles, such as for instance magnetic particles, whereby the other antibody has a group resembling a detectable label or enabling for selective attachment to a label, and which serves the detection of the formed sandwich structures. A temporarily delayed or subsequent immobilization using suitable solid phases is also possible.

The method according to the present invention can furthermore be embodied as a homogeneous method, wherein the sandwich complexes formed by the antibody/antibodies and the marker, e.g., copeptin, which is to be detected remains suspended in the liquid phase. In this case it is preferred, that when two antibodies are used, both antibodies are labeled with parts of a detection system, which leads to generation of a signal or triggering of a signal if both antibodies are integrated into a single sandwich. Such techniques are to be embodied in particular as fluorescence enhancing or fluorescence quenching detection methods. A particularly preferred aspect relates to the use of detection reagents which are to be used pairwise, such as for example the ones which are described in US 4 882 733 A, EP-B1 0 180 492 or EP-B1 0 539 477 and the prior art cited therein. In this way, measurements in which only reaction products comprising both labeling components in a single immune-complex directly in the reaction mixture are detected, become possible. For example, such technologies are offered under the brand names TRACE^{®} (Time Resolved Amplified Cryptate Emission) or KRYPTOR^{®}, implementing the teachings of the above-cited applications. Therefore, in particular preferred aspects, a diagnostic device is used to carry out the herein provided method. For example, copeptin and/or the level of any further marker of the herein provided method is determined.

In a preferred aspect, both the first and the second antibody are dispersed in the liquid reaction medium, whereby a first labeling component which is part of a labeling system based on fluorescence- or chemoluminescence quenching or enhancement is bound to the first antibody, and whereby the second labeling component of this labeling system is bound to the second antibody, such that after binding of both antibodies to copeptin, a detectable signal is generated which enables for a detection of the sandwich complexes formed in the measuring solution. One aspect of this alternative comprises the labeling system such as rare earth cryptates or chelates in combination with a fluorescence- or cheminoluminescence-dye. In a particular preferred aspect, the labeling system comprises a rare earth cryptate in combination with a fluorescence or chemiluminescence dye, in particular, of the cyanine type. In a further preferred aspect, the detection is carried out with a competitive immunoassay. In a preferred aspect, a radioimmunoassay is used. It also envisaged herein that the level of the marker can be, for example, determined by mass spectrometric methods or by a high performance liquid chromatography (HPLC) method, which can be coupled to an immunoassay, or a massspectrometric based approach. The skilled person understands that any available assay can be used as long as the level of the marker can be reliably determined.

In one aspect, the method is an immunoassay comprising the steps of:
a) contacting the sample with
   (i) a first antibody or an antigen-binding fragment or derivative thereof specific for a first epitope of copeptin, and
   (ii) a second antibody or an antigen-binding fragment or derivative thereof specific for a second epitope of copeptin; and
b) detecting the binding of the first and second antibodies or antigen-binding fragments or derivates thereof to copeptin.

Typically, in this context one of the antibodies is labeled and the other antibody is bound to a solid phase or can be bound selectively to a solid phase. For example, the first antibody and the second antibody are present dispersed in a liquid reaction mixture, and wherein a first labelling component which is part of a labelling system based on fluorescence or chemiluminescence extinction or amplification is bound to the first antibody, and a second labelling component of said labelling system is bound to the second antibody so that, after binding of both antibodies to copeptin, a measurable signal which permits detection of the resulting sandwich complexes in the measuring solution is generated.

As indicated herein above, labelling systems may comprise a rare earth cryptate or chelate in combination with a fluorescent or chemiluminescent dye, in particular of the cyanine type.

In principle, all labeling techniques which can be applied in assays of said type can be used, such as labeling with radioisotopes, enzymes, fluorescence-, chemoluminescence- or bioluminescence labels and directly optically detectable color labels, such as gold atoms and dye particles, which are used in particular in Point-of-Care (POC) or rapid tests. In the case of heterogeneous sandwich immunoassays, both antibodies may exhibit parts of the detection system according to the type described herein in the context of homogenous assays.

Moreover, mass spectrometry approaches can be used to detect and quantify copeptin. In this context, in one aspect of the present invention, copeptin can also be determined by a mass spectrometric (MS) based method. Herein, the term "mass spectrometry" or "MS" refers to an analytical method to identify compounds by their mass. Such a method may comprise detecting the presence, amount or concentration of one or more modified or unmodified fragment peptides of copeptin in said biological sample or a protein digest (e.g. tryptic digest) from said sample, and optionally separating the sample with chromatographic methods, and subjecting the prepared and optionally separated sample to MS analysis. For example, selected reaction monitoring (SRM), multiple reaction monitoring (MRM) or parallel reaction monitoring (PRM) mass spectrometry may be used in the MS analysis, particularly to determine the amounts of copeptin.

In order to enhance the mass resolving and mass determining capabilities of mass spectrometry, the samples can be processed prior to MS analysis. Accordingly, the invention relates to MS detection methods that can be combined with immunoenrichment technologies, methods related to sample preparation and/or chromatographic methods, preferably with liquid chromatography (LC), more preferably with high performance liquid chromatography (HPLC) or ultra high performance liquid chromatography (UHPLC). Sample preparation methods comprise techniques for lysis, fractionation, digestion of the sample into peptides, depletion, enrichment, dialysis, desalting, alkylation and/or peptide reduction. However, these steps are optional. The selective detection of analyte ions may be conducted with tandem mass spectrometry (MS/MS). Tandem mass spectrometry is characterized by mass selection step (as used herein, the term "mass selection" denotes isolation of ions having a specified m/z or narrow range of m/z's), followed by fragmentation of the selected ions and mass analysis of the resultant product (fragment) ions. The skilled person is aware of assays that are suitable to determine/quantify the herein described markers. The skilled person is aware how quantify the level of a marker in the sample by mass spectrometric methods. Moreover, the levels (including cut-off levels) can be determined by mass spectrometric based methods, such as methods determining the relative quantification or determining the absolute quantification of the protein or fragment thereof of interest.

In the context of the present invention, the term "treatment" refers to a medical treatment and comprises various treatments and therapeutic strategies, which comprise, without limitation, administration of analogue(s) of vasopressin.

As used in accordance with the methods of the present invention, the drug for the treatment of MEN used is preferably Desmopressin. Desmopressin (1-desamino-8-D-arginine vasopressin, dDAVP) is an analogue of vasopressin. Desmopressin has decreased vasopressor activity and increased anti-diuretic activity compared to vasopressin. This pharmacological profile enables Desmopressin to be used clinically for anti-diuresis without causing significant increases in blood pressure. Desmopressin is commercially available as the acetate salt both in tablet form and as a nasal spray, and is commonly prescribed for primary nocturnal enuresis (PNE) and central diabetes insipidus. Desmopressin may be administered intravenously, subcutaneously, intranasally, and orally. Intranasal administration of Desmopressin via a nasal spray is approved for the maintenance treatment of patients with central diabetes insipidus and in children (ages 6 to 16 years) with primary nocturnal enuresis. An oral tablet dosage form of Desmopressin has also been approved for the treatment of central diabetes insipidus and primary nocturnal enuresis. In patients with nocturnal enuresis, particularly MEN, the anti-diuretic effect of Desmopressin decreases urine volume at night, lowering the amount of urine which the urinary bladder must retain and, thereby decreasing or eliminating occurrences of enuresis. Preferably, Desmopressin is administered in the form of a tablet. More preferably, Desmopressin is administered in the form of a sublingual tablet. It may be formulated as a pharmaceutically acceptable salt, e.g. as Desmopressin acetate.

In another aspect of the present invention, Desmopressin is typically applied in a dose of 40 to 400 µg, e.g. 100 µg, 200 µg, 400 µg or preferably of about 120 µg per day.

### Sequences

SEQ ID NO:1 (amino acid sequence of pre-pro-AVP): 
1 
51 
101 
151 
SEQ ID NO:2 (amino acid sequence of AVP):
   1 CYFQNCPRG
SEQ ID NO:3 (amino acid sequence of CT-pro-AVP (copeptin)):
   1 ASDRSNATQL DGPAGALLLR LVQLAGAPEP FEPAQPDAY
SEQ ID NO:4 (amino acid sequence of neurophysin II):

1 AMSDLELRQC LPCGPGGKGR CFGPSICCAD ELGCFVGTAE ALRCQEENYL
51 PSPCQSGQKA CGSGGRCAAF GVCCNDESCV TEPECREGFH RRA

The following non-binding Example illustrates the invention.

### Example

### Synopsis - Copeptin for prediction of treatment response in children with monosymptomatic nocturnal enuresis (MEN).

The Example has been performed by detecting copeptin.

### Study population

100 children with monosymptomatic nocturnal enuresis. Exclusion criteria consider factors with known or possible effect on copeptin levels or functional disorders of the urinary tract.

### Eligibility criteria

### Inclusion criteria:

Children at the age between 5 and 16 years
Diagnosis of monosymptomatic nocturnal enuresis
Completion of uroflowmetry and bladder sonography if possible
Completion of home recording charts of bed wetting episodes if possible
Willingness to use Desmopressin treatment

### Exclusion criteria:

Children with daytime enuresis
Children with lower urinary tract symptoms and infection
Children with structural abnormalities of the urinary tract
Children with chronic illness, esp. renal failure, hypertension, congenital heart disease, diabetes mellitus and diabetes insipidus.

### Hypothesis

The withinday copeptin amplitude at baseline predicts the successful treatment at the end of therapy.

### Primary and Secondary Objective

The primary objective of the first study part is to investigate whether the amplitude of the whithinday copeptin change at baseline (i.e. difference of copeptin between morning and evening) predicts treatment response to Desmopressin after 4 weeks of treatment.

The primary objective of the second study part is to estimate the predictive accuracy of the withinday copeptin amplitude with respect to a successful Desmopressin treatment response.

### Endpoints

Primary endpoint: Successful response to Desmopressin treatment after 4 weeks, defined as 14 consecutive dry nights or a 90% improvement in the number of wet nights per week (9).

Secondary endpoint: Treatment response to Desmopressin after 8 weeks in patients with dose escalation to 240 µg Desmopressin.

### Study design

This is a prospective, observational two-phases study in 100 children with MEN. Patients go through 5 visits with each visit having a duration of approx. 30 minutes. 2 of these visits take place at the patient's home (Table 1).

### Management of participants throughout the study

**Table 1: Visit and assessment schedule**

| | Enrolment day | Examination day | | | | |
|---|---|---|---|---|---|---|
| | | Day 1 in hospital | Day 1 at home | After 4 weeks in hospital | After 4 weeks at home | After 12 weeks in hospital |
| | | At 8am | At 8(10)pm | At 8 am | At 8(10) pm | At 8 am |
| Informed consent | X | | | | | |
| Eligibility criteria | X | | | | | |
| Medical history questionnaire | X | | | | | |
| Diary completed | X | | | X | | X |

| Clinical parameters | | | | | | |
|---|---|---|---|---|---|---|
| Body temperature (C°) | X | X | X | X | X | X |
| Blood pressure (mmHg) | X | X | X | X | X | X |
| Heart rate | X | X | X | X | X | X |

| Examination parameters | | | | | | |
|---|---|---|---|---|---|---|
| Bladder sonography | X | | | | | |
| Uroflow (if possible) | X | | | | | |

| Laboratory parameters | | | | | | |
|---|---|---|---|---|---|---|
| Copeptin | X | X | X | X | X | |

### Study sequence in detail

### At Enrolement day:

1. All patients, or their legal representatives respectively, are asked to sign the informed consent form.
2. After subscription eligibility criteria will be assessed
3. The 1 week diary for bedwetting is completed.
4. A standardized medical history questionnaire with focus on MEN will be conducted.

### At examination day:

All participants arrive to the ward in the morning of the examination day at 8am for the first blood sample before starting the Desmopressin treatment with sublingual tablets of 120 µg. The diary is handed out to the study nurse or study doctor. At the same day a study nurse visits the patient at its home between 8 and 10 pm for the second blood sample immediately before the first intake of the sublingual tablet before bedtime. After 4 weeks of Desmopressin treatment the patient visits the clinic of pediatric urology and another blood sample is taken at 8 am. At the same day, a study nurse visits the patients at home and takes a blood sample for copeptin between 8 and 10 pm before bedtime.

If the patient did not respond satisfyingly to Desmopressin treatment the dose is increased to 240 µg sublingual tablet per day. All patients are seen 12 weeks after start of Desmopressin therapy. Prior to the visit all patients fulfill the diary for 1 week. With every visit clinical parameters are taken as they are body temperature, blood pressure and heart rate.

All blood tests are taken by capillary blood sampling.

### End of study:

The study ends 12 weeks after start with 120 µg Desmopressin.

### Blood sampling

At every time point 1 tube is collected. For capillary withdrawal 500 µl blood is needed for every blood sampling. In case of all 4 visits the patient give 2000 µl blood (i.e. 2ml). Therefore the estimated blood loss is much below the limit for permitted blood withdrawal in children (9). Copeptin is measured with a chemiluminiscence sandwich immunoassay with a lower detection limit of the assay of 0.4pmol/l.

### Assessment of safety-potential risks and adverse events

Desmopressin can lead to side effects like hyponatremia, water intoxication and hypertension especially in very high dose treatment and extensive water intake. The administration of sublingual tablets of 120 µg, resp. 240 µg, has shown to have a good safety profile (10.11). The estimated Blood loss is. 2 ml in 12 weeks which is much below the limit for permitted blood withdrawal in children (9). All recommendations outlined in the ICH Guidelines for Good Clinical Practice are adhered to throughout this trial.

### Statistical methology, sample size consideration and data processing

The aim of the study is to investigate whether the withinday copeptin amplitude at baseline (before start of Desmopressin therapy) can predict the successful treatment at the end of therapy. In the second phase of the study the estimated baseline copeptin amplitude that predicts the chance of a successful Desmopressin treatment response will be confirmed. A logistic regression model is used to investigate whether the withinday copeptin amplitude is explaining the outcome of successful treatment.

All relevant clinical and laboratory parameters obtained by interview, clinical tests and blood sampling are entered into a secuTrial database. Copeptin levels are assessed in batch analysis upon completion of the aliquoted plasma samples. Discrete variables are expressed as counts (percentage) and continuous variables as means ± standard deviation (SD) or median (interquartile range), unless stated otherwise.

For the first phase of the study, sample size was calculated to be able to show that the withinday copeptin amplitude at baseline can be used to predict the successful treatment outcome (= reduction in bed-wetting of 90 %).

A logistic regression model is used to investigate whether the withinday copeptin amplitude is explaining the outcome successful treatment. Sample size was set to ensure at least 90 % power ( 1 - β = 0.9) at a significance level of α = 5 %. Assuming that 30 % of the patients are treated successfully, 52 patients should be recruited to ensure 41 evaluable patients considering a drop-out rate of 20 %.

The second phase of the study calculates the ppV of the estimated withinday copeptin amplitude at baseline using the second half of the 100 patients. Detailed methodology is described in the analysis plan.

All relevant clinical, laboratory parameters and exam results are entered into an Excel^{®} database. Voluntary participants who do not fulfill eligibility criteria are replaced. Participants may withdraw from the study at any time. Reasons for withdrawal are documented. Results are summarized using all available data. Multiple attempts are made to obtain missing data, but missing data is not imputed.

### Results

The trial is analysed in two stages: The data of the first part of the patients (within day copeptin amplitude and success of Desmopressin therapy after 12 weeks) is used to confirm the logistic model and to estimate an ideal cut-off. The cut-off of the baseline copeptin amplitude that predicts the chance of a successful Desmopressin treatment response is calculated. The data of the second study part is used to estimate the predictive accuracy together with a 95 % confidence interval.

**Table 2: Copeptin to predict treatment response with minirin in patients with enuresis nocturna**

| | **No treatment response** | **Treatment response** |
|---|---|---|
| **Participants** | 30% (6) | 70% (14) |
| **Morning Copeptin** | median 7.6 pg/ml (IQR 4.7-10.8) | median 5.9 pg/ml (IQR 4.8-8.2) |
| **Evening Copeptin** | median 7.7 pg/ml (IQR 7-10) | median 5.7 pg/ml (IQR 4.3-7.1) |
| **Ratio Copeptin (morning/evening)** | median 0.95 (IQR 0.6-1.5) | median 1.14 (IQR 0.9-1.4) |
| **Delta Copeptin (morning-evening)** | median -0.05 pg/ml (IQR -3.3-3.35) | median 0.6 pg/ml (IQR -0.5-1.8) |

## Claims

1. A method for predicting whether a subject to be treated for monosymptomatic nocturnal enuresis will respond to said treatment comprising the steps of
(i) determining a first level (c1) of copeptin in a first sample of said subject which has been obtained at a first time,
(ii) determining a second level (c2) of copeptin in a second sample of said subject which has been obtained at a second time,
wherein said first time is after an overnight fast of minimum 8 hrs and between 6 a.m. and 9 a.m. in the morning and said second time is between 6 pm and 9 p.m. at the same day, and
wherein if the ratio of said first level to said second level (c1 / c2) is ≥ 1.00, said subject is predicted to respond to said treatment.

2. The method of claim 1 wherein said treatment is a treatment with Desmopressin or any derivatives thereof.

3. The method of any of the preceding claims, wherein said subject is between 5 and 17 years of age, preferably between 5 and 16 years of age, more preferably between 6 and 16 years of age, more preferably between 6 and 11 years of age.

4. The method of any one of the preceding claims, wherein said first and second samples are samples of a bodily fluid, preferably blood, plasma or serum, more preferably plasma or serum.

5. The method of any one of the preceding claims, wherein said level of copeptin is determined using a method selected from the group consisting of Luminescenceimmunoassay (LIA), radioimmunoassay (RIA), chemiluminescence- and fluorescence- immunoassay, enzyme immunoassay (EIA), Enzyme-linked immunoassay (ELISA), luminescence-based bead array, magnetic beads based array, protein microarray assay, rapid test formats, and rare cryptate assay.

6. The method of any one of the preceding claims, wherein the method is an immunoassay comprising the steps of:
a) contacting the first and second samples with
(i) a first antibody or an antigen-binding fragment or derivative thereof specific for a first epitope of copeptin, and
(ii) a second antibody or an antigen-binding fragment or derivative thereof specific for a second epitope of copeptin; and
b) detecting the binding of the first and second antibodies or antigen-binding fragments or derivates thereof to copeptin.

7. The method of claim 6, wherein the first antibody and the second antibody are present dispersed in a liquid reaction mixture, and wherein a first labelling component which is part of a labelling system based on fluorescence or chemiluminescence extinction or amplification is bound to the first antibody, and a second labelling component of said labelling system is bound to the second antibody so that, after binding of both antibodies to copeptin, a measurable signal which permits detection of the resulting sandwich complexes in the measuring solution is generated.

8. Desmopressin for use in the treatment of monosymptomatic nocturnal enuresis, wherein Desmopressin is administered to a subject that has been determined to be responsive in a method according to claims 1 to 8.

## Patentansprüche

1. Verfahren zum Vorhersagen, ob ein Subjekt, das wegen monosymptomatischer Enuresis noctura behandelt werden soll, auf die Behandlung ansprechen wird, umfassend die Schritte
(i) Bestimmen eines ersten Spiegels (c1) von Copeptin in einer ersten Probe des Subjekts, die zu einem ersten Zeitpunkt erhalten wurde,
(ii) Bestimmen eines zweiten Spiegels (c2) von Copeptin in einer zweiten Probe des Subjekts, die zu einem zweiten Zeitpunkt erhalten wurde,
wobei der erste Zeitpunkt nach einem Fasten über Nacht von mindestens 8 Stunden und zwischen 6 Uhr und 9 Uhr morgens liegt und der zweite Zeitpunkt zwischen 18 Uhr und 21 Uhr an dem selben Tag liegt, und
wobei, falls das Verhältnis des ersten Spiegels zu dem zweiten Spiegel (c1 / c2) ≥ 1,00 ist, vorhergesagt wird, dass das Subjekt auf die Behandlung anspricht.

2. Verfahren nach Anspruch 1, wobei die Behandlung eine Behandlung mit Desmopressin oder einem beliebigen Derivat davon ist.

3. Verfahren nach einem der vorstehenden Ansprüche, wobei das Subjekt zwischen 5 und 17 Jahre alt, vorzugsweise zwischen 5 und 16 Jahre alt, mehr bevorzugt zwischen 6 und 16 Jahre alt, noch mehr bevorzugt zwischen 6 und 11 Jahre alt, ist.

4. Verfahren nach einem der vorstehenden Ansprüche, wobei die erste und die zweite Probe Proben eines Körperfluids, vorzugsweise Blut, Plasma oder Serum, mehr bevorzugt Plasma oder Serum, sind.

5. Verfahren nach einem der vorstehenden Ansprüche, wobei der Spiegel von Copeptin unter Verwendung eines Verfahrens bestimmt wird, das aus der Gruppe ausgewählt ist, bestehend aus Lumineszenzimmunoassay (LIA), Radioimmunoassay (RIA), Chemilumineszenz- und Fluoreszenzimmunoassay, Enzymimmunoassay (EIA), Enzyme-gekoppeltem Immunoassay (ELISA), Kügelchen-Array auf Lumineszenzbasis, Array auf Basis magnetischer Kügelchen, Protein-Microarray-Assay, Schnelltestformaten und seltenem Kryptat-Assay.

6. Verfahren nach einem der vorstehenden Ansprüche, wobei das Verfahren ein Immunoassay ist, umfassend die Schritte:
a) Kontaktieren der ersten und der zweiten Probe mit
(i) einem ersten Antikörper oder einem Antigen-bindenden Fragment oder einem Derivat davon, das für ein erstes Epitop des Copeptin spezifisch ist, und
(ii) einem zweiten Antikörper oder einem Antigen-bindenden Fragment oder einem Derivat davon, das für ein zweites Epitop des Copeptin spezifisch ist; und
b) Nachweisen der Bindung des ersten und des zweiten Antikörpers oder der Antigen-bindenden Fragmente oder Derivate davon an Copeptin.

7. Verfahren nach Anspruch 6, wobei der erste Antikörper und der zweite Antikörper dispergiert in einem flüssigen Reaktionsgemisch vorhanden sind, und wobei eine erste Markierungskomponente, die Teil eines Markierungssystems basierend auf Fluoreszenz- oder Chemilumineszenzextinktion oder -verstärkung ist, an den ersten Antikörper gebunden ist, und eine zweite Markierungskomponente des Markierungssystems an den zweiten Antikörper gebunden ist, sodass nach der Bindung beider Antikörper an Copeptin, ein messbares Signal erzeugt wird, das einen Nachweis der resultierenden Sandwichkomplexe in der Messlösung zulässt.

8. Desmopressin zur Verwendung bei der Behandlung von monosymptomatischer Enuresis noctura, wobei Desmopressin einem Subjekt verabreicht wird, bei dem bestimmt wurde, dass es in einem Verfahren nach den Ansprüchen 1 bis 8 darauf anspricht.

## Revendications

1. Procédé permettant de prédire si un sujet devant être traité pour une énurésie nocturne monosymptomatique répondra audit traitement, comprenant les étapes consistant à
(i) déterminer un premier niveau (c1) de copeptine dans un premier échantillon dudit sujet, qui a été obtenu à un premier moment,
(ii) déterminer un second niveau (c2) de copeptine dans un second échantillon dudit sujet, qui a été obtenu à un second moment,
dans lequel le premier moment se situe après un jeûne d'une nuit d'au moins 8 heures et entre 6 et 9 heures du matin et le second moment entre 18 et 21 heures le même jour, et
dans lequel, si le rapport entre ledit premier et ledit second niveau (c1 / c2) est ≥ 1,00, il est prévu que ledit sujet réponde audit traitement.

2. Procédé selon la revendication 1, dans lequel le traitement est un traitement à la Desmopressine ou à l'un quelconque des dérivés de celui-ci.

3. Procédé selon l'une quelconque des revendications précédentes, dans lequel le sujet est âgé de 5 à 17 ans, préférablement de 5 à 16 ans, plus préférablement de 6 à 16 ans, plus préférablement de 6 à 11 ans.

4. Procédé selon l'une quelconque des revendications précédentes, dans lequel les premier et second échantillons sont des échantillons d'un fluide corporel, de préférence du sang, du plasma ou du sérum, plus préférablement du plasma ou du sérum.

5. Procédé selon l'une quelconque des revendications précédentes, dans lequel ledit niveau de copeptine est déterminé à l'aide d'un procédé choisi dans le groupe constitué par un dosage immunologique par luminescence (LIA), un dosage immunologique par radio-immunité (RIA), un dosage immunologique par chimiluminescence et fluorescence, un dosage immunologique enzymatique (EIA), un dosage immunologique par absorption enzymatique (ELISA), un réseau de billes à luminescence, un réseau de billes magnétiques, un dosage de microréseaux de protéines, des formats d'essai rapide et un dosage de cryptats rares.

6. Procédé selon l'une quelconque des revendications précédentes, dans lequel le procédé est un dosage immunologique comprenant les étapes consistant à :
a) mettre en contact les premier et second échantillons avec
(i) un premier anticorps ou fragment de liaison à un antigène ou un dérivé de celui-ci spécifique pour un premier épitope de copeptine, et
(ii) un second anticorps ou fragment de liaison à un antigène ou un dérivé de celui-ci spécifique pour un second épitope de copeptine ; et
b) détecter la liaison des premier et second anticorps ou fragments de liaison à l'antigène ou dérivés de ceux-ci à la copeptine.

7. Procédé selon la revendication 6, dans lequel le premier anticorps et le second anticorps sont présents dispersés dans un mélange réactionnel liquide, et dans lequel un premier composant de marquage qui fait partie d'un système de marquage basé sur l'extinction ou l'amplification par fluorescence ou chimiluminescence est lié au premier anticorps, et un second composant de marquage dudit système de marquage est lié au second anticorps de telle sorte que, après liaison des deux anticorps à la copeptine, un signal mesurable qui permet la détection des complexes sandwich résultant dans la solution de mesure est généré.

8. Desmopressine à utiliser dans le traitement de l'énurésie nocturne monosymptomatique, dans laquelle la desmopressine est administrée à un sujet qui a été déterminé comme étant réactif dans un procédé selon les revendications 1 à 8.
